(19) Europäisches Patentamt
European Patent Office
Office européen des brevets

(11) Veröffentlichungsnummer : **0 016 346**
**B1**

(12) # EUROPÄISCHE PATENTSCHRIFT

(45) Veröffentlichungstag der Patentschrift : **03.02.82**

(51) Int. Cl.³ : **C 07 C125/065**, C 07 C125/073

(21) Anmeldenummer : **80100874.9**

(22) Anmeldetag : **22.02.80**

(54) **Verfahren zur Herstellung von Urethanen.**

(30) Priorität : **02.03.79 DE 2908251**

(43) Veröffentlichungstag der Anmeldung : **01.10.80 (Patentblatt 80/20)**

(45) Bekanntmachung des Hinweises auf die Patenterteilung : **03.02.82 Patentblatt 82/05**

(84) Benannte Vertragsstaaten : **AT BE CH DE FR GB IT NL**

(56) Entgegenhaltungen :
**DE - A1 - 2 603 574**
**EP - A1 - 563**

**CHEMISTRY LETTERS, 1972, Tokyo K. KONDO et al. : « A New Synthesis of Carbamates. The Reaction of Carbon Monoxide with Amine and Alcohol in the Co-Presence of Selenium and Triethylamine », Seiten 373 bis 374**

(73) Patentinhaber : **BAYER AG**
**Zentralbereich Patente, Marken und Lizenzen**
**D-5090 Leverkusen 1, Bayerwerk (DE)**

(72) Erfinder : **Becker, Robert, Dr.**
**Fichtestrasse 2 a**
**D-5090 Leverkusen (DE)**
Erfinder : **Grolig, Johann, Dr.**
**Heinrich-Lübke-Strasse 22**
**D-5090 Leverkusen (DE)**
Erfinder : **Rasp, Christian, Dr.**
**Wolfskaul 10**
**D-5000 Köln 80 (DE)**

**EP 0 016 346 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von Urethanen

Die vorliegende Erfindung betrifft ein verbessertes Verfahren zur Herstellung von Urethanen durch Umsetzung von primären Aminen mit Kohlenmonoxid und mindestens eine Hydroxylgruppe aufweisenden organischen Verbindungen.

Organische Isocyanate werden großtechnisch im allgemeinen durch Umsetzung der entsprechenden Amine mit Phosgen hergestellt. Wegen der Giftigkeit des Phosgens ist man seit geraumer Zeit bestrebt, einen großtechnisch gangbaren Syntheseweg zu organischen Isocyanaten aufzufinden, bei welchem sich die Verwendung des Phosgens erübrigt. Ein derartiger Syntheseweg besteht in der Umsetzung von organischen Nitroverbindungen mit Kohlenmonoxid und organischen Hydroxylverbindungen zu den entsprechenden Urethanen und deren anschließende Spaltung in Isocyanat und Hydroxylgruppen aufweisende Verbindungen, wobei vor der Spaltung auch eine Modifizierung des als Zwischenprodukt erhaltenen Urethans denkbar ist. So ist es beispielsweise möglich, das aus Nitrobenzol, Kohlenmonoxid und Äthanol zugängliche Phenylurethan zunächst mit Formaldehyd zum Bisurethan des 4,4'-Diisocyanatodiphenylmethans umzusetzen, um das so erhaltene Zwischenprodukt unter Abspaltung des Äthanols in 4,4'-Diisocyanatodiphenylmethan zu überführen.

Die Spaltung von Urethanen in die entsprechenden Isocyanate und Hydroxylgruppen aufweisenden Verbindungen ist beispielsweise in der DE-OS 2 421 503 bzw. in den in dieser Literaturstelle abgehandelten Vorveröffentlichungen beschrieben.

Für die Herstellung der Urethane sind in der Patentliteratur die Umsetzung von Nitroverbindungen mit Kohlenmonoxid und Alkoholen in Gegenwart von Selen oder Selenverbindungen (DE-OS 2 343 826, 2 614 101, 2 623 694) oder von Edelmetallen, insbesondere Palladium, in Gegenwart von Lewissäuren (DE-OS 1 568 044 und 2 603 574) beschrieben. Diese Umsetzung verläuft im Falle der Veroendung einer Mononitroverbindung nach folgender Stöchiometrie :

$$R - NO_2 + 3CO + R'OH \longrightarrow RNHCO_2R' + 2CO_2$$

Die allgemeine Reaktionsgleichung lautet :

$$R(NO_2)_x + 3xCO + xR'OH \longrightarrow R(NHCO_2R')_x + 2xCO_2.$$

Das heißt, daß pro Mol an zu erzeugender Urethangruppe 3 Mole Kohlenmonoxid verbraucht und 2 Mole Kohlendioxid gebildet werden. Das eingesetzte Kohlenmonoxid wird somit nur zu einem Drittel für die Herstellung der Urethangruppierung genutzt, während zwei Drittel in das technisch wertlose inerte Kohlendioxid umgewandelt werden. Bedingt durch die hohe Wärmetönung der exothermen Bildung von $CO_2$ sind bei der technischen Durchführung der bekannten Urethan-Synthese auf der Basis von Nitroverbindung, Kohlenmonoxid und Alkohol aufwendige Vorrichtungen zur Abführung der hohen Reaktionswärme nötig.

Aus Chemistry Letters (Chemical Society of Japan), Jahrgang 1972, Seiten 373-374 ist weiterhin bekannt, primäre Amine mit stöchiometischen Mengen Selen, einem tertiären Amin, Kohlenmonoxid und Alkohol zu einem Komplexsalz umzusetzen, aus welchem man mit molekularem Sauerstoff das entsprechende Urethan erhält und Selen sowie tertiäres Amin zurückgewinnt :

$$RNH_2 + Se + R''_3N + CO + R'OH \rightarrow \left[R''_3NH\right]^{\oplus} \left[RNH\overset{OR'}{\underset{OH}{C}}Se\right]^{\ominus} \quad (I)$$

$$I + 1/2O_2 \rightarrow RNH\overset{O}{\overset{\|}{C}}OR' + Se + R''_3N$$

Als Bruttoreaktion erfolgt eine Urethan-Bildung nach folgender Gleichung :

$$RNH_2 + CO + R'OH + 1/2O_2 \longrightarrow RNHCO_2R' + H_2O$$

wobei nur noch 1 Mol CO je zu erzeugende Urethangruppe benötigt wird. Entsprechend ist die exotherme Reaktionswärme geringer, da nur noch 1 Mol Wasser anstelle von 2 Mol Kohlendioxid bei Einsatz von Nitroverbindungen gebildet wird. Für eine technische Urethan-Synthese ist jedoch diese mit stöchiometrischen Mengen an Selen « katalysierte » Oxycarbonylierung ungeeignet, da untragbar hohe Mengen des teuren und giftigen Selens eingesetzt werden müssen, dessen quantitative Rückgewinnung aus dem Reaktionsgemisch schwierig ist. Die unter Verwendung von Selen durchgeführte Oxycarbonylierung muß in 2 Stufen durchgeführt werden, was die technische Durchführung weiterhin erschwert. Die erhaltenen Ausbeuten sind bei dieser Arbeitsweise unbefriedigend.

Es wurde nun gefunden, daß es möglich ist, eine Oxycarbonylierung von primären Aminen zu Urethanen in technisch brauchbaren Ausbeuten unter Einsparung von Kohlenmonoxid und verbesserter Wärmeabführung durchzuführen, ohne die für die Arbeitsweise mit Selen sich ergebenden Nachteile in Kauf nehmen zu müssen, wenn man primäre Amine mit Kohlenmonoxid und mindestens eine Hydroxylgruppe aufweisenden organischen Verbindungen in Gegenwart von molekularem Sauerstoff und/oder organischen Nitroverbindungen als Oxidationsmittel und in Gegenwart bestimmter, nachstehend näher beschriebener Katalysatoren zur Reaktion bringt.

Die EP-A 1-563 beschreibt zwar die Herstellung von Urethanen durch Umsetzung von aromatischen Nitroverbindungen mit Kohlenoxid und Hydroxylgruppen aufweisenden Verbindungen in Gegenwart von Edelmetallkatalysatoren, Lewis-Säuren und primären Aminen, wobei das Molverhältnis zwischen primären Aminen und Nitroverbindungen im weiten Bereich von 0,1:1 bis 100:1 liegen kann, jedoch wird an keiner Stelle nahegelegt, daß die Verwendung von Aminen als Ausgangsmaterialien und von Nitroverbindungen als Oxydationsmittel in Gegenwart der nachstehend näher beschriebenen Katalysatorkomponenten bb) in irgendeiner Weise vorteilhaft sein könnte. In der Tat werden in den Ausführungsbeispielen der Vorveröffentlichung entweder überhaupt keine Aminoverbindungen oder nur untergeordnete Mengen an Aminoverbindungen im Vergleich zu den Nitroverbindungen eingesetzt. Ein Hinweis auf die ganz speziellen, nachstehend näher beschriebenen Katalysatorkomponenten bb) fehlt völlig. Andererseits gestattet das nachstehend näher beschriebene erfindungsgemäße Verfahren, und hierin ist der Hauptvorteil gegenüber dem Verfahren der Vorveröffentlichung zu sehen, eine optimale Ausbeute bezogen auf das eingesetzte Kohlenoxid. Bei einem Verfahren, bei welchem Nitroverbindungen als wesentliche Ausgangsmaterialien eingesetzt werden, wie dies in allen Ausführungsbeispielen der Vorveröffentlichung der Fall ist, treten zwangsläufig die bereits obengenannten Nachteile auf. Trotz des lapidaren Hinweises auf das zitierte breite Molverhältnis zwischen Aminogruppen und Nitrogruppen kann gesagt werden, daß die Vorveröffentlichung keinen richtungsweisenden Hinweis vermittelt, daß diese, auch in allen Ausführungsbeispielen der Vorveröffentlichung auftretenden Nachteile durch das nachstehend näher beschriebene erfindungsgemäße Verfahren beseitigt werden könnten.

Gegenstand der vorliegenden Erfindung ist ein Verfahren zur Herstellung von Urethanen durch Umsetzung von primären Aminen mit Kohlenmonoxid und mindestens eine Hydroxylgruppe enthaltenden organischen Verbindungen, dadurch gekennzeichnet, daß man die Umsetzung in Gegenwart von

a) molekularem Sauerstoff und/oder organischen Nitroverbindungen als Oxidationsmittel und

b) einem Katalysatorsystem durchführt, welches

ba) aus einem Edelmetall und/oder einer Edelmetallverbindung der achten Nebengruppe des Periodensystems der Elemente und

bb) unter den Reaktionsbedingungen zu Redoxreaktionen befähigten, von den Verbindungen ba) verschiedenen Verbindungen von Elementen der dritten bis fünften Hauptgruppe und/oder der ersten bis achten Nebengruppe des Periodensystems der Elemente besteht, wobei im Falle der Verwendung von organischen Nitroverbindungen als Komponente a) als Komponente bb) Oxychloride von unter den Reaktionsbedingungen zu Redoxreaktionen befähigten Elementen der dritten bis fünften Hauptgruppe oder ersten bis achten Nebengruppe des Periodensystems oder Kombinationen von (i) Oxiden und/oder Hydroxyden von unter den Reaktionsbedingungen zu Redoxreaktionen geeigneten Elementen der dritten bis fünften Hauptgruppe oder ersten bis achten Nebengruppe des Periodensystems mit (ii) anionisch, als Chlorid gebundenes Chlor enthaltenden Verbindungen, die unter den Reaktionsbedingungen zur Aktivierung der Oxide bzw. Hydroxyde unter Chloridbildung geeignet sind, zum Einsatz gelangen.

Ausgangsmaterialien für das erfindungsgemäße Verfahren sind beliebige primäre Amine mit mindestens einer aliphatisch, cycloaliphatisch und/oder aromatisch gebundenen Aminogruppe eines im allgemeinen zwischen 31 und 400, vorzugsweise 93 bis 279, liegenden Molekulargewichts, sowie beliebige mindestens eine Hydroxygruppe enthaltende organische Verbindung, beispielsweise substituierte, aliphatische, cycloaliphatische und/oder aromatische Mono- oder Polyhydroxyverbindungen eines im allgemeinen zwischen 32 und 300, vorzugsweise 32 und 102, liegenden Molekulargewichts.

Beispielsweise können folgende aromatischen primären Amine eingesetzt werden: Anilin, 1,2-Diaminobenzol, 1,3-Diaminobenzol, 1,4-Diaminobenzol, o-Chlor-anilin, m-Chloranilin, Toluylidin, Xylidine, 2,3-Diamino-toluol, 2,4-Diamino-toluol, 2,6-Diamino-toluol, 2,5-Diamino-toluol, 3,4-Diamino-toluol, 3,5-Diamino-toluol, Diaminoxylole, 1-Aminonaphthalin, 2-Aminonaphthalin, Diamino-naphthalin, Aminoanthracene, 4,4'-Diaminodiphenylmethan, 2,2'-Diamino-diphenylmethan, 2,4'-Diamino-diphenylmethan oder Tris-(4-aminophenyl)-methan.

Als cycloaliphatische primäre Amine seien genannt:

Aminocyclobutan, Aminocyclopentan, Cyclohexylamin, 1,2-Diaminocyclohexan, 1,3-Diaminocyclohexan, 1,4-Diaminocyclohexan, Bis-(aminocyclohexyl)-methane, Tri-(aminocyclohexyl)-methane.

Beispielhaft für die Gruppe der aliphatischen primären Amine seien genannt: Methylamin, Ethylamin, 1-Propylamin, 2-Propylamin, 1-Butylamin, 2-Butylamin, Isobutylamin, tert.-Butylamin, 1-Pentylamin, 1-Hexylamin, 1-Heptylamin, 1-Octylamin, 1-Decylamin, 1-Dodecylamin, Äthylendiamin, 1,2-Diaminopropan, 1,3-Diaminopropan, Diaminobutane, Diaminopentane, Diaminohexane, Diaminooctane, Diaminodecane, Benzylamin, Bis-(aminomethyl)-cyclohexane, Bis-(aminomethyl)-benzol, ω-Aminocarbonsäureester, ω-Aminocarbonsäurenitrile.

Besonders bevorzugte primäre Amine sind aromatische primäre Amine wie insbesondere Anilin, 1,3-Diaminobenzol, 2,4-Diaminotoluol, 2,6-Diaminotoluol oder 1,5-Diaminophthalin.

Ausgangsmaterialien für das erfindungsgemäße Verfahren sind desweiteren beliebige Hydroxylgruppen aufweisende organische Verbindungen wie z.B. beliebige ein- oder mehrwertige Alkohole bzw. ein-oder mehrwertige phenole. Geeignete Alkohole sind beispielsweise solche des Molekulargewichtsbereichs 32-300. Es kann sich bei diesen Alkoholen um beliebige lineare oder verzweigte ein- oder mehrwertige Alkanole oder Alkenole, um beliebige ein- oder mehrwertige Cycloalkanole, Cycloalkenole oder Aralkylalkohole handeln. Auch beliebige inerte Substituenten aufweisende Alkohole sind geeignet. Geeignete Substituenten sind z.B. Halogenatome, Sulfoxidgruppen, Sulfongruppen, Carbonyl- oder Carbonsäureestergruppen. Auch Etherbrücken aufweisende Alkohole sind grundsätzlich geeignet. Beispiele geeigneter Alkohole sind : Methanol, Ethanol, n-Propanol, Isopropanol, n-Butanol, n-Pentanol, n-Hexanol, Cyclohexanol, Benzylalkohol, Chlorethanol, Ethylenglycol, Diethylenglykol, Propylenglykol, Dipropylenglykol, Glycerin, Hexantriol oder Trimethylolpropan. Bevorzugt werden einwertige aliphatische Alkohole mit 1 bis 6 Kohlenstoffatomen eingesetzt.

Geeignete Phenole sind insbesondere solche eines Molekulargewichtsbereichs 94-300 wie beispielsweise Phenol, die isomeren Chlorphenole, Kresole, Ethylphenole, Propylphenole, Butylphenole oder höheren Alkylphenole, Brenzcatechin, 4,4'-Dihydroxydiphenylmethan, Bisphenol-A, Anthranol, Phenanthrol, Pyrogallol oder Phloroglucin. Die beispielhaft genannten Alkohole sind gegenüber den beispielhaft genannten Phenolen bevorzugt. Ethanol ist die besonders bevorzugte beim erfindungsgemäßen Verfahren einzusetzende Hydroxylgruppen aufweisende Verbindung.

Bei der Durchführung des erfindungsgemäßen Verfahrens werden die organischen Hydroxylverbindungen im allgemeinen in solchen Mengen eingesetzt, daß für jedes Mol an im Reaktionsgemisch vorliegenden primären Aminogruppen und für jedes Mol an gegebenenfalls im Reaktionsgemisch vorliegenden Nitrogruppen 1 bis 100, vorzugsweise 1 bis 20 Mol an Hydroxylgruppen vorliegen. Da im allgemeinen unter den Reaktionsbedingungen flüssige Hydroxylverbindungen zum Einsatz gelangen, dient deren Überschuß als Reaktionsmedium (Lösungsmittel) für das erfindungsgemäße Verfahren.

Als weiterer Reaktionspartner kommt beim erfindungsgemäßen Verfahren Kohlenmonoxid zum Einsatz. Dieses Ausgangsmaterial wird im allgemeinen in einer Menge eingesetzt, die 1 bis 30 Mol Kohlenmonoxid pro Mol zu erzeugendem Urethan entspricht, d.h. es kommen für jedes Mol an im Reaktionsgemisch vorliegenden primären Aminogruppen und für jedes Mol an gegebenenfalls im Reaktionsgemisch vorliegenden Nitrogruppen 1 bis 30 Mol Kohlenmonoxid zum Einsatz.

Die erfindungsgemäße Umsetzung erfolgt in Gegenwart von a) Oxidationsmitteln und b) Katalysatoren.

Als Oxidationsmittel kann molekularer Sauerstoff in reiner Form oder in Form von Gesmischen mit Inertgas, wie Stickstoff oder Kohlendioxid, insbesondere in Form von Luft, eingesetzt werden. In Gegenwart von molekularem Sauerstoff erfolgt die Oxycarbonylierung nach folgender allgemeiner Reaktionsgleichung :

$$R(NH_2)_x + \frac{x}{2}O_2 + xCO + xR'OH \longrightarrow R(NHCO_2R')_x + xH_2O,$$

das heißt, je gebildeter Urethangruppe wird nur 1 Mol Kohlenmonoxid benötigt.

Der als Oxidationsmittel verwendete molekulare Sauerstoff kann im Unterschuß eingesetzt werden. Bevorzugt wird ein Inertgas wie Stickstoff oder Kohlendioxid zugegeben, wobei die Mengen so bemessen werden, daß die Reaktion außerhalb des Explosionsbereichs der Sauerstoff-CO-Gemische, bzw. der Sauerstoff-Alkohol-Gemische, durchgeführt werden kann. Ohne Inertgaszugabe ist die Sauerstoffzugabe so zu bemessen, daß explosible Gemische mit dem Kohlenmonoxid und der Alkohol-Komponente vermieden werden. Bevorzugt wird der molekulare Sauerstoff in Form von Luft oder Luft-Stickstoffgemischen eingesetzt. Im Falle der Verwendung von molekularem Sauerstoff als alleinigem Oxidationsmittel wird dessen Menge vorzugsweise so bemessen, daß, entsprechend obiger Reaktionsgleichung, für jedes Mol an primären Aminogruppen mindestens ein halbes Mol Sauerstoff zur Verfügung steht, wobei selbstverständlich auch überschüssige Mengen an Sauerstoff eingesetzt werden können.

Bevorzugte Oxidationsmittel sind organische Nitroverbindungen. Man kann eine im Vergleich zum eingesetzten primären Amin in der Struktur vollkommene andersartige Nitroverbindung einsetzen, wobei man sowohl aus dem eingesetzten primären Amin als auch aus der Nitroverbindung Urethane erhält, die sich jedoch in der Struktur unterscheiden. Die Oxycarbonylierung erfolgt in diesem Falle beispielsweise nach folgender Reaktionsgleichung :

$$2R^1(NH_2)_x + R^2(NO_2)_x + 3xCO + 3xR'OH \longrightarrow$$

$$2R^1(NHCO_2R')_x + R^2(NHCO_2R')_x + 2xH_2O$$

Im Interesse einer möglichst optimalen Ausbeute werden bei Verwendung von organischen Nitroverbindungen als alleinigem Oxidationsmittel die Mengen des primären Amins und der Nitroverbindung vorzugsweise so bemessen, daß im Reaktionsgemisch auf jede Nitrogruppe zwei Aminogruppen entfallen. Bei einem Unterschuß an Nitroverbindungen findet nur ein unvollständiger Aminumsatz statt, während bei einem stöchiometrischen Überschuß an Nitrogruppen diese nach der Gleichung :

$$R(NO_2)_x + 3xCO + xR'OH \longrightarrow R(NHCO_2R')_x + 2xCO_2$$

unter Bildung von Kohlendioxid zur Urethangruppe umgesetzt werden, wodurch die besonders vorteilhafte Ausnutzung des Kohlenmonoxid wieder teilweise verlorengeht.

Diese Mengenverhältnisse sind jedoch selbstverständlich nicht zwingend einzuhalten, insbesondere kann bei gleichzeitiger Mitverwendung von molekularem Sauerstoff auch eine geringere Menge an Nitroverbindung zum Einsatz gelangen. Ganz allgemein kann gesagt werden, daß die Nitroverbindungen in solchen Mengen zugegeben werden, daß das Äquivalentverhältnis der Aminogruppen der primären Amine zu den Nitrogruppen zwischen 1,1:1 und 4:1, insbesondere zwischen 1,5:1 und 2,5:1 und ganz besonders bevorzugt zwischen 1,8:1 bis 2,2:1 liegt.

Eine besonders vorteilhafte Variante des erfindungsgemäßen Verfahrens besteht darin, daß man Nitroverbindungen einsetzt, die bezüglich ihrer Konstitution dem primären Amin entsprechen, so daß aus 2 Mol primären Amin und einem Mol Nitroverbindung 3 Mol Urethan der gleichen Struktur entsprechend folgender Reaktionsgleichung erhalten werden :

$$2R(NH_2)_x + R(NO_2)_x + 3xCO + 3xR'OH \longrightarrow$$

$$3R(NHCO_2R')_x + 2xH_2O.$$

Für das erfindungsgemäße Verfahren geeignete Nitroverbindungen sind beliebige Nitrogruppen aufweisende, organische Verbindungen mit mindestens einer aliphatisch, cycloaliphatisch und/oder aromatisch gebundenen Nitrogruppe, eines im allgemeinen zwischen 61 und 400, vorzugsweise 123 und 262 liegenden Molekulargewichts.

Beispielsweise können folgende aromatische Nitroverbindungen eingesetzt werden : Nitrobensol, o-Dinitrobenzol, m-Dinitrobenzol, p-Dinitrobenzol, o-Chlornitrobenzol, m-Chlor-nitrobenzol, o-Chlor-nitrobenzol, o-Nitrotoluol, m-Nitrotoluol, p-Nitrotoluol, 2,3-Dinitrotoluol, 2,4-Dinitrotoluol, 2,5-Dinitrotoluol, 2,6-Dinitrotoluol, 3,4-Dinitrotoluol, 3-Nitro-o-xylol, 4-Nitro-o-xylol, 2-Nitro-m-xylol, 4-Nitro-m-xylol, 5-Nitro-m-xylol, Nitro-p-xylol, 3,4-Dinitro-o-xylol, 3,5-Dinitro-o-xylol, 3,6-Dinitro-o-xylol, 4,5-Dinitro-o-xylol, 2,4-Dinitro-m-xylol, 2,5-Dinitro-m-xylol, 4,5-Dinitro-m-xylol, 4,6-Dinitro-m-xylol, 2,3-Dinitro-p-xylol, 2,6-Dinitro-p-xylol, 1-Nitronaphthalin, 2-Nitronaphthalin, Dinitronaphthaline, Nitroanthracene, Nitrodiphenyle, Bis-(nitrophenyl)-methane, Bis-(nitrophenyl)-thioäther, Bis-(nitrophenyl)-sulfone, Nitrodiphenoxyalkane, Nitrophenothiazine.

Als cycloaliphatische Nitroverbindungen seien genannt : Nitrocyclobutan, Nitrocyclopentan, Nitrocyclohexan, 1,2-Dinitrocyclohexan, 1,3-Dinitrocyclohexan, 1,4-Dinitrocyclohexan, Bis-(nitrocyclohexyl)-methane.

Beispielhaft für die Gruppe der Nitroalkane seien genannt : Nitromethan, Nitroäthan, 1-Nitropropan, 2-Nitropropan, Nitrobutane, Nitropentane, Nitrohexane, Nitrodecane, Nitrocetane, 1,2-Dinitroäthan, 1,2-Dinitropropan, 1,3-Dinitropropan, Dinitrobutane, Dinitropentane, Dinitrohexane, Dinitrodecane, Phenylnitromethan, Bis-(nitromethyl)-cyclohexane, Bis-(nitromethyl)-benzole, ω-Nitrocarbonsäurenitrile.

Bevorzugte Nitroverbindungen für das erfindungsgemäße Verfahren sind aromatische Nitroverbindungen der oben beispielhaft genannten Art. Besonders bevorzugte Nitroverbindungen sind Nitrobenzol, 1,3-Dinitrobenzol, 2,4-Dinitrotoluol, 2,6-Dinitrotoluol oder z.B. 1,5-Dinitronaphthalin.

Die beim erfindungsgemäßen Verfahren zum Einsatz gelangenden Katalysatorensysteme b) enthalten als wesentlichen Bestandteil ba) Edelmetalle der achten Nebengruppe des Periodensystems und bb) eine Cokatalysator-Komponente.

Bei der Katalysatorenkomponente ba) handelt es sich entweder um freie Edelmetalle der achten Nebengruppe des Periodensystems oder um im Reaktionsgemisch lösliche Verbindungen dieser Metalle. Besonders vorteilhaft werden die Edelmetalle als im Reaktionsgemisch lösliche Verbindungen zugegeben, beispielsweise als Chloride, Bromide, Jodide, Chlorokomplexe, Bromokomplexe, Jodokomplexe, Acetate, Acetylacetonate u.a. lösliche Edelmetallverbindungen. Bevorzugte Edelmetalle sind Palladium und Rhodium. Besonders bevorzugt wird Palladium, insbesondere in Form von löslichem Palladiumchlorid, eingesetzt. Die bevorzugten Konzentrationen, bezogen auf das Reaktionsgemisch inklusive gegebenenfalls mitverwendetem Lösungsmittel liegen im allgemeinen bei 0,000 1 bis 0,1 Gew.-%, insbesondere 0,001 bis 0,01 Gew.-%, berechnet als Edelmetall. Bei niedrigeren Edelmetallkonzentrationen wird die Reaktionsgeschwindigkeit zu gering. Höhere Edelmetall-Konzentrationen sind zwar möglich, jedoch wegen der möglichen Edelmetallverluste unwirtschaftlich, da ohnehin eine weitere Steigerung der Urethanausbeuten nicht mehr erfolgt.

Bei den Cokatalysatoren bb) handelt es sich im Falle der ausschließlichen Verwendung von molekularem Sauerstoff als Komponente a) um beliebige unter den Reaktionsbedingungen zu Redoxreaktionen befähigte, von der Komponente ba) verschiedene Verbindungen von unter den Reaktionsbedingungen zu Redoxreaktionen befähigten Elementen der 3. bis 5. Haupt- und 1. bis 8. Nebengruppe des

Periodensystems. Die entsprechenden Verbindungen stellen vorzugsweise Chloride, Oxichloride, Oxide und/oder Hydroxide der genannten Elemente dar, wobei im Falle des Einsatzes der entsprechenden Oxide bzw. Hydroxide vorzugsweise bestimmte aktivierend wirkende Chloride mitverwendet werden.

Geeignete Cokatalysatoren sind beispielsweise Kupfer (II)-chlorid, Thallium (III)-chlorid, Zinn-(II)-chlorid, Zinn-(IV)-chlorid, Arsen (III)-chlorid, Bismut (III)-chlorid, Vanadin (III)-chlorid, Chrom (III)-chlorid, Molybdän (IV)-chlorid, Wolfram (V)-chlorid, Wolfram (VI)-chlorid, Mangan (II)-chlorid, Eisen (II)-chlorid, Eisen (III)-chlorid, Eisenoxichlorid, Kobalt (II)-chlorid, Kupfer (II)-oxid, Kupfer (II)-hydroxid, Thallium (I)-hydroxid, Zinn (II)-oxid, Zinn (II)-hydroxid, Vanadinpentoxid, Molybdäntrioxid, Wolframtrioxid, Mangandioxid, Eisen (II)-oxid, Eisen (II)-hydroxid, Eisen (III)-hydroxid, Eisen (III)-oxide, wie z.B. $\alpha$-$Fe_2O_3$, $\gamma$-$Fe_2O_3$, Eisen (III)-oxidhydrate, wie z.B. $\alpha$-FeO-OH, $\beta$-FeO-OH, $\gamma$-FeO-OH und Eisenspinell $Fe_3O_4$.

Zu den besonders bevorzugten Cokatalysatoren gehören Eisen (II)-chlorid, Eisen (III)-chlorid, Eisenoxichlorid, sowie die Oxide und Oxidhydrate des dreiwertigen Eisens.

Im Falle der Verwendung der beispielhaft genannten, unter den Reaktionsbedingungen oft völlig inerten Oxide bzw. Hydroxide ist die Mitverwendung von aktivierenden Chloriden erforderlich. Es handelt sich hierbei um anionisch, als Chlorid gebundenes Chlor enthaltende Verbindungen, die unter den Reaktionsbedingungen mit den beispielhaft genannten Oxiden bzw. Hydroxiden unter deren zumindest teilweisen Überführung in die entsprechenden Chloride bzw. Oxychloride zu reagieren vermögen. Geeignete aktivierende Chloride sind beispielsweise Hydrochloride von tertiären Aminen des Molekulargewichtsbereichs 59 bis 300, Hydrochloride der beim erfindungsgemäßen Verfahren eingesetzten primären Amine, Chlorwasserstoff, Eisen (II)-chlorid oder Eisen (II)-chlorid-Komplexe. Besonders gut geeignete aktivierende Chloride sind Pyridinhydrochlorid, Anilinhydrochlorid, das Hydrochlorid von 2,4-Diaminotoluol, Chlorwasserstoff, Eisen (II)-chlorid oder Eisen (II)-chlorid-Komplexe. Kombinationen der zuletzt genannten besonders bevorzugten aktivierenden Chloride mit den oxiden und Oxidhydraten des dreiwertigen Eisen stellen besonders wertvolle Cokatalysatoren bb) dar.

Im Falle der Verwendung von organischen Nitroverbindungen als Komponente a) werden als Komponente bb) zwingend Oxichloride von unter den Reaktionsbedingungen zu Redoxreaktionen befähigten Elementen der dritten bis fünften Hauptgruppe oder ersten bis achten Nebengruppe des Periodensystems oder Kombinationen von (i) Oxiden und/oder Hydroxyden von unter den Reaktionsbedingungen zu Redoxreaktionen geeigneten Elementen der dritten bis fünften Hauptgruppe oder ersten bis achten Nebengruppe des Periodensystems mit (ii) anionisch, als Chlorid gebundenes Chlor enthaltenden Verbindungen, die unter den Reaktionsbedingungen zur Aktivierung der Oxide bzw. Hydroxyde unter Chloridbildung geeignet sind, verwendet.

Die Cokatalysatoren einschließlich der aktivierenden Chloride werden beim erfindungsgemäßen Verfahren im allgemeinen in Konzentrationen von 0,1 bis 20 Gew.-%, vorzugsweise 1 bis 5 Gew.-%, bezogen auf Reaktionsgemisch inklusive gegebenenfalls mitverwendeten Lösungsmittel eingesetzt. Der Anteil der aktivierenden Chloride liegt, falls diese überhaupt erforderlich sind, hierbei im allgemeinen bei 0,05 bis 10, vorzugsweise 0,1-bis 2,5 Gew.-%.

Die erfindungsgemäße Umsetzung kann in Gegenwart oder in Abwesenheit eines Lösungsmittels durchgeführt werden. Im allgemeinen dient die vorzugsweise im Überschuß eingesetzte organische Hydroxylverbindung als Lösungsmittel. Die Mitverwendung inerter Lösungsmittel, die bis zu 80 Gew.-% des gesamten Reaktionsansatzes ausmachen können, ist jedoch auch möglich. Die Menge des Lösungsmittels muß, gleichgültig ob es sich um im Überschuß eingesetzte Hydroxylverbindung oder um inertes Lösungsmittel handelt, so bemessen sein, daß die Reaktionswärme der exothermen Urethanbildung ohne unzulässige Temperaturerhöhung abgeführt werden kann. Im allgemeinen wird daher das erfindungsgemäße Verfahren unter Verwendung einer Konzentration von Aminoverbindungen von 5-50 Gew.-%, vorzugsweise 5-20 Gew.-% und bei Einsatz von organischen Nitroverbindungen als Oxidationsmittel unter Verwendung einer Konzentration an Nitroverbindungen von 1 bis 20 Gew.-%, bevorzugt von 5 bis 10 Gew.-%, bezogen auf das Gesamtreaktionsgemisch inklusive dem Lösungsmittel durchgeführt.

Brauchbare Lösungsmittel sind gegen die Reaktionskomponenten und das Katalysatorsystem inerte Lösungsmittel, wie beispielsweise aromatische, cycloaliphatische und aliphatische Kohlenwasserstoffe, die gegebenenfalls durch Halogen substituiert sein können, wie Benzol, Toluol, Xylol, Chlorbenzol, Dichlorbenzol, Trichlorbenzol, Chlornaphthalin, Cyclohexan, Methylcyclohexan, Chlorcyclohexan, Methylenchlorid, Tetrachlorkohlenstoff, Tetrachloräthan, Trichlor-trifluoräthan u.ä. Verbindungen.

Die Reaktionstemperatur liegt im allgemeinen zwischen 100 °C und etwa 300 °C, insbesondere zwischen 130 °C und 250 °C und besonders vorteilhaft im Bereich von 140 °C bis 220 °C. Der Druck muß so bemessen sein, daß stets das Vorliegen einer flüssigen Phase gewährleistet ist und liegt im allgemeinen im Bereich von 5 bis 500 bar, besonders vorteilhaft im Bereich von 30 bis 300 bar bei Reaktionstemperatur. Je nach eingesetztem primären Amin bzw. Hydroxyverbindung und gegebenenfalls organischer Nitroverbindung beträgt die für den quantitativen Umsatz benötigte Reaktionszeit zwischen wenigen Minuten und mehreren Stunden.

Die Oxycarbonylierung der primären Amine mit den Hydroxyverbindungen, Kohlenmonoxid und dem Oxidationsmittel zu Urethanen kann diskontinuierlich oder kontinuierlich durchgeführt werden.

Die diskontinuierliche Umsetzung kann im Hochdruckautoklaven mit geringen Mengen homogen gelöstem Edelmetall und einem ausreichenden Überschuß Katalysator, gegebenenfalls in Gegenwart

eines aktivierenden Chlorids, durchgeführt werden. Im Reaktionsmedium unlösliche Verbindungen, wie beispielsweise Eisenoxide, bzw. Eisenoxidhydrate, können in Form eines feinen Pulvers zugegeben werden, die aktivierenden Zusatzstoffe in Form einer homogenen alkoholischen Lösung. Die Verteilung nicht gelöster überschüssiger Cokatalysatorbestandteile kann durch kräftiges Rühren oder durch Umpumpen des Reaktionsgemisches erfolgen. Die exotherme Reaktionswärme kann durch intern eingebaute Kühlaggregate oder im Falle des Umpumpens auch über einen externen Wärmeaustauscher abgeführt werden. Die Aufarbeitung und Katalysatorrückführung kann je nach Löslichkeit des erzeugten Urethans im Reaktionsgemisch auf verschiedene Weise erfolgen. Bei leichtlöslichen Urethanen kann beispielsweise nach beendeter Reaktion die Hauptmenge des bei tiefen Temperaturen schwerlöslichen Cokatalysatorgemisches samt dem größten Teil des adsorbierten Palladiums und des organischen Aminsalzes vom Reaktionsprodukt abgetrennt werden, beispielsweise durch Filtration oder Zentrifugieren, und in eine neue Umsetzung von primären Aminen Hydroxylverbindungen, Kohlenmonoxid und Oxidationsmittel wieder zurückgeführt werden. Das flüssige Reaktionsgemisch kann auf übliche Weise, z.B. durch fraktionierte Destillation, in Lösungsmittel, in die reinen Urethane und gegebenenfalls kleine Mengen von Nebenprodukten aufgetrennt werden, wobei diese Trennung diskontinuierlich oder kontinuierlich erfolgen kann. Im Destillationsrückstand sind kleine Mengen der im Reaktionsgemisch gelösten Cokatalysatorbestandteile und/oder Spuren von Edelmetall und/oder Edelmetallverbindungen enthalten, die wieder in die Umsetzung zurückgeführt werden können.

Im Falle von im Lösungsmittel bzw. überschüssiger Hydroxyverbindung schwer löslichen Urethanen kann die Aufarbeitung des Reaktionsgemisches in abgeänderter Weise erfolgen. Beispielsweise wird nach Entspannung unter Druck und höherer Temperatur, bei welchen die Urethane noch gelöst sind, jedoch das Katalysatorsystem Edelmetall/Cokatalysatorgemisch weitgehend ausfällt, die Hauptmenge Katalysator abfiltriert oder abzentrifugiert und dann durch Temperaturerniedrigung das schwerlösliche Urethan, gegebenenfalls zusammen mit geringen Mengen schwerlöslicher Nebenprodukte und restlichem Katalysator, auskristallisiert. Die Mutterlauge, die außer Lösungsmittel, bzw. der als Lösungsmittel angewandten überschüssigen organischen Hydroxyverbindung, noch geringe Mengen Nebenprodukte, gelöstes Urethan und gegebenenfalls gelöste Cokatalysatorbestandteile enthält, kann direkt oder nach vorheriger Entfernung leichtsiedender Nebenprodukte, beispielsweise durch Destillation, in die Oxycarbonylierung der primären Amine mit den Hydroxyverbindungen, Kohlenmonoxid und dem Oxidationsmittel zurückgeführt werden, wobei die dem vorherigen Umsatz entsprechende Menge primäres Amin, Hydroxyverbindung und gegebenenfalls Nitroverbindung als Oxidationsmittel, ergänzt wird. Höhersiedende Nebenprodukte, welche nicht durch Kristallisation entfernt werden, können durch destillative Aufarbeitung eines aliquoten Teiles der Mutterlauge als Destillationsrückstand kontinuierlich aus dem Rückführstrom entfernt werden. Das ausgefallene Rohurethan kann beispielsweise durch Kristallisation aus einem das Urethan bei höheren Temperaturen lösenden, die Nebenprodukte und die Katalysatorreste jedoch nicht lösenden Lösungsmittel wie beispielsweise Isooktan, Benzol, Toluol, Xylol, Chlorbenzol, Dichlorbenzol rekristallisiert werden. Die bei erhöhter Temperatur unlöslichen Rückstände können durch Oxidation in unlösliche Oxide, wie beispielsweise Eisenoxide, und ein aus den organischen Verunreinigungen resultierendes Abgas, das im wesentlichen aus Kohlendioxid, Sauerstoff, Stickstoff und gegebenenfalls leicht flüchtigen organischen Verunreinigungen besteht, umgewandelt werden. Das Abgas kann je nach Zusammensetzung direkt in die Atmosphäre abgelassen werden oder zusätzlich einer katalytischen Nachverbrennung zugeführt werden, in welcher restliche Verunreinigungen oxidativ entfernt werden. Die aus dem Rückstand erhaltene oxidische Verbindung, die gegebenenfalls noch geringe Mengen Edelmetall und/oder Edelmetallverbindung enthalten kann, wird wieder in die Oxycarbonylierung zurückgeführt.

Das bei der Oxycarbonylierung erhaltene Reaktionsgas, welches nicht umgesetztes Kohlenmonoxid, leichtsiedende organische Bestandteile, geringe Mengen Kohlendioxid und bei Verwendung von molekularem Sauerstoff als Oxidationsmittel auch geringe Mengen nicht umgesetzten Sauerstoff, sowie das zusätzlich mit eingebrachte Inertgas, wie beispielsweise Stickstoff, enthalten kann, wird beispielsweise nach Abtrennung der leichtsiedenden organischen Nebenprodukte und gegebenenfalls von Kohlendioxid, wieder auf Reaktionsdruck gebracht und in die Reaktion zurückgeführt unter Ergänzung der verbrauchten Anteile Kohlenmonoxid und gegebenenfalls molekularen Sauerstoff.

Die kontinuierliche Umsetzung kann in einer Kesselkaskade, einem Rohrbündelreaktor, mehreren hintereinander geschalteten Schlaufenreaktoren, in einem oder einer Serie hintereinander geschalteten adiabaten Reaktionsrohren oder in einer Blasensäule durchgeführt werden. Die Wärmeabführung erfolgt beispielsweise entweder intern durch eingebaute Kühlaggregate, extern über einen Rohrbündelwärmetauscher oder adiabatisch über die Wärmekapazität des Reaktionsgemisches mit nachfolgender Abkühlung in externen Kühlaggregaten.

Die weitere Aufarbeitung kann wie oben beschrieben erfolgen, wobei sowohl eine kontinuierliche als auch eine diskontinuierliche Arbeitsweise angewandt werden kann.

Im Falle der bevorzugten Verwendung der erfindungsgemäßen Verfahrensprodukte als Zwischenprodukte zur Herstellung der entsprechenden Isocyanate ist ihre Reindarstellung oft überflüssig. Zur weiteren Verarbeitung kann es vielmehr ausreichend sein, die nach Abfiltrieren des Katalysators und gegebenenfalls Abdestillieren des Lösungsmittels anfallende Rohprodukte in die Weiterverarbeitung einzusetzen.

Das Verfahren wird durch die folgenden Beispiele illustriert, ohne damit die Erfindung auf die in den Beispielen gegebenen Bedingungen einzuschränken.

## Beispiele

### Beispiel 1

In einem 0,7 l Edelstahlautoklaven werden 250 g (270 ml) eines Gemischs folgender Zusammensetzung vorgelegt : $2.10^{-3}$ Gew.-% Palladiumchlorid, 4 Gew.-% Eisenoxychlorid, 5 Gew.-% Anilin und 91 Gew.-% Äthanol. Bei Raumtemperatur werden sodann 100 bar Kohlenmonoxid und 25 bar Luft aufgepreßt, auf 150 °C aufgeheizt und 2 Stunden bei dieser Temperatur reagieren gelassen. Nach Abkühlen auf Raumtemperatur wird entspannt und die flüssige Phase sowie die Gasphase gaschromatografish analysiert. Es errechnet sich ein Anilin-Umsatz von 77 %, sowie folgende Phenylurethan-Selektivitäten : 90 %, bezogen auf Anilin, 95 %, bezogen auf Äthanol, und 78 %, bezogen auf Kohlenmonoxid, wobei die Selektivität auf Kohlenmonoxid hier und in den folgenden Beispielen nach der Stöchiometrie 1 Mol CO je 1 Urethangruppe berechnet wurde.

### Beispiel 2

Es wurde wie in Beispiel 1 gearbeitet, jedoch anstelle von Palladiumchlorid Rhodiumchlorid eingesetzt. Nach 2 Stunden Reaktionszeit bei 180 °C waren 50 % des Anilins umgesetzt. Phenylurethan wurde in einer Selektivität von 60 %, bezogen auf Anilin und von 90 %, bezogen auf Äthanol, erhalten.

### Beispiel 3

Es wurde im wesentlichen wie in Beispiel 1 gearbeitet, jedoch als Kokatalysator ein Gemisch aus 4 Gew.-% $\alpha$-$Fe_2O_3$ und 1 Gew.-% $FeCl_2 . 4H_2O$ eingesetzt. Nach 1 Stunde Reaktionszeit bei 180 °C waren 60 % des Anilins umgesetzt. Die Urethan-Selektivitäten betrugen 75 %, bezogen auf Anilin, 94 %, bezogen auf Äthanol und 88 %, bezogen auf Kohlenmonoxid.

### Beispiel 4

Zu 250 g (270 ml) eines Gemischs aus $2. 10^{-3}$ Gew.-% Palladiumchlorid, 2,8 Gew.-% $MnO_2$, 1,9 Gew.-% HCl (wasserfrei) und 5 Gew.-% Anilin in Äthanol wurden in einem 0,7 l-Autoklaven bei 20 °C 100 bar Kohlenmonoxid und 25 bar Luft aufgepreßt, auf 180 °C schnell aufgeheizt und 1 Stunde bei dieser Temperatur belassen. Danach wurde auf 40 °C abgekühlt, entspannt und nochmals mit der gleichen Menge Kohlenmonoxid und Luft bei 180 °C 1 Stunde umgesetzt. Man erhielt einen Anilin-Umsatz von 78 % und Phenylurethan-Selektivitäten von 71 %, bezogen auf Anilin, 99 %, bezogen auf Äthanol und 76 %, bezogen auf Kohlenmonoxid.

### Beispiel 5

In einem 0,7 l Edelstahlautoklaven wurden 250 g eines Gemischs aus $2 . 10^{-3}$ Gew.-% Palladiumchlorid, 4 Gew.-% Eisenoxychlorid und 20 Gew.-% eines Gemisches aus Anilin und Nitrobenzol in Äthanol mit dem Molverhältnis Anilin : Nitrobenzol = 2:1 vorgelegt und bei Raumtemperatur 120 bar Kohlenmonoxid aufgepreßt. Es wurde auf 160 °C schnell hochgeheizt und bei dieser Temperatur 2 Stunden belassen. Nach Abkühlen und Entspannen ergab die gaschromatographische Analyse folgende Resultate : Anilin war zu 85 %, Nitrobenzol quantitativ umgesetzt worden. Die Phenylurethan-Selektivitäten betrugen 96 %, bezogen auf Anilin und Nitrobenzol, 99,3 %, bezogen auf Äthanol und 77 %, bezogen auf Kohlenmonoxid.

### Beispiel 6

Der Versuch von Beispiel 5 wurde mit folgenden Änderungen wiederholt : Anstelle von Äthanol wurde Methanol eingesetzt und die Reaktionsdauer betrug 5 Stunden. Der Anilin-Umsatz errechnete sich zu 97 %, der Nitrobenzol-Umsatz zu 92 %. Man erhielt 85 % N-Phenyl-methylurethan, bezogen auf umgesetztes Anilin und Nitrobenzol, und 93 %, bezogen auf umgesetztes Kohlenmonoxid.

### Beispiele 7 bis 10

In der folgenden Tabelle sind Oxycarbonylierungen von Anilin mit Nitrobenzol in Äthanol mit verschiedenen Kokatalysatoren aufgeführt.

Allgemeine Bedingungen :

| | |
|---|---|
| $PdCl_2$ | $2 . 10^{-3}$ Gew. % |
| Konzentration von Anilin + Nitrobenzol in Äthanol | 20 Gew. % |

| | |
|---|---|
| Molverhältnis Anilin : Nitrobenzol | 2 : 1 |
| Temperatur °C | 180 |
| Druck bar | 120 bei 20 °C |
| Reaktionszeit h | 1 |

Ergebnisse :

| Beispiel Nr. | Kokatalysator (Gew.-%) | Umsätze % Anilin | Nitro-benzol | Phenylurethan-Selektivität % auf Anilin + Nitrob. | Äthanol | CO |
|---|---|---|---|---|---|---|
| 7 | $CuCl_2$ (3,3) + $V_2O_5$ (2) | 75 | 100 | 85 | 95 | 95 |
| 8 | $\alpha$-$Fe_2O_3$ (2) + $FeCl_2 \cdot 4H_2O$ (0,6) | 80 | 100 | 96 | 99 | 90 |
| 9 | $\alpha$-$Fe_2O_3$ (2,8) + Anilin · HCl (3,6) | 90 | 100 | 90 | 99 | 85 |
| 10 | $V_2O_5$ (2,8) + Anilin · HCl (3,6) | 80 | 100 | 98 | — | 85 |

Beispiel 11

Zu 250 g eines Gemischs aus 2 . $10^{-3}$ Gew.-% Palladiumchlorid, 3,7 Gew.-% Eisenoxychlorid, 7,4 Gew.-% Anilin und 3,0 Gew.-% 2,4-Dinitrotoluol in Äthanol wurden bei Raumtemperatur in einem 0,7 l-Autoklaven 120 bar Kohlenmonoxid aufgepreßt und 2 Stunden bei 180 °C umgesetzt. Das Anilin wurde zu 90 % umgesetzt. Man errechnete eine Phenylurethan-Selektivität von 93 %, bezogen auf Anilin.

Beispiel 12

Zu 250 g eines Gemischs aus 2 . $10^{-3}$ Gew.-% Palladiumchlorid, 2,7 Gew.-% $\alpha$-$Fe_2O_3$, 0,8 Gew.-% $FeCl_2 . 4 H_2O$, 4,5 Gew.-% 2,4-Diaminotoluol und 6,5 Gew.-% 2,4-Dinitrotoluol in Äthanol wurden bei Raumtemperatur in einem 0,7 l-Autoklaven 120 bar Kohlenmonoxid aufgepreßt und 2 Stunden bei 180 °C umgesetzt. Sowohl das 2,4-Diaminotoluol als auch das 2,4-Dinitrotoluol waren quantitativ umgesetzt. Man erhielt folgende Selektivitäten, bezogen auf die Summe Diamin + Dinitroverbindungen : 75 % Bisurethan des 2,4-Diisocyanatotoluols, 15 % Monourethan-Gemisch, 7 % Nitroaminotoluole.

Beispiel 13

Dieses Beispiel illustriert die katalytische Wirksamkeit von Rhodiumverbindungen bei der Oxycarbonylierung der primären Amine mit Nitroverbindungen. Es wurden 250 g eines Reaktionsgemischs folgender Zusammensetzung vorgelegt : 2,7 . $10^{-3}$ Gew.-% Rhodiumtrichlorid, 3,7 Gew.-% Eisenoxychlorid, 11,6 Gew.-% Anilin und 7,67 Gew.-% Nitrobenzol. Bei Raumtemperatur wurden in einem 0,7 l-Autoklaven 120 bar Kohlenmonoxid aufgepreßt und sodann 1 Stunde bei 180 °C umgesetzt. Es wurden 59 % des Anilins und 60 % des Nitrobenzols umgesetzt. Die Phenylurethanausbeute, bezogen auf die Summe von umgesetzten Anilin + Nitrobenzol, errechnete sich aus den gaschromatographischen Analysen zu 85 Mol-%.

**Ansprüche**

1. Verfahren zur Herstellung von Urethanen durch Umsetzung von primären Aminen mit Kohlenmonoxid und mindestens eine Hydroxylgruppe enthaltenden organischen Verbindungen, dadurch gekennzeichnet, daß man die Umsetzung in Gegenwart von
a) molekularem Sauerstoff und/oder organischen Nitroverbindungen als Oxidationsmittel und
b) einem Katalysatorsystem durchführt, welches
ba) aus einem Edelmetall und/oder einer Edelmetallverbindung der achten Nebengruppe des Periodensystems der Elemente und
bb) unter den Reaktionsbedingungen zu Redoxreaktionen befähigten, von den Verbindungen ba) verschiedenen Verbindungen von Elementen der dritten bis fünften Hauptgruppe und/oder der ersten bis achten Nebengruppe des Periodensystems der Elemente besteht, wobei im Falle der Verwendung von organischen Nitroverbindungen als Komponente a) als Komponente bb) Oxychloride von unter den Reaktionsbedingungen zu Redoxreaktionen befähigten Elementen der dritten bis fünften

Hauptgruppe oder ersten bis achten Nebengruppe des Periodensystems oder Kombinationen von (i) Oxiden und/oder Hydroxyden von unter den Reaktionsbedingungen zu Redoxreaktionen geeigneten Elementen der dritten bis fünften Hauptgruppe oder ersten bis achten Nebengruppe des Periodensystems mit (ii) anionisch, als Chlorid gebundenes Chlor enthaltenden Verbindungen, die unter den Reaktionsbedingungen zur Aktivierung der Oxide bzw. Hydroxyde unter Chloridbildung geeignet sind, zum Einsatz gelangen.

2. Verfahren gemäß Anspruch 1, dadurch gekennzeichnet, daß man als organische Nitroverbindung die dem eingesetzten primären Amin konstitutionell entsprechende Nitroverbindung verwendet.

3. Verfahren gemäß Anspruch 1 bis 2, dadurch gekennzeichnet, daß man als Katalysatorkomponente ba) Palladium, Rhodium, Palladiumverbindungen und/oder Rhodiumverbindungen verwendet.

4. Verfahren gemäß Anspruch 1 bis 3 unter Verwendung von molekularem Sauerstoff als Komponente a), dadurch gekennzeichnet, daß man als Katalysatorkomponente bb) Oxichloride von unter den Reaktionsbedingungen zu Redoxreaktionen befähigten Elementen der dritten bis fünften Hauptgruppe oder ersten bis achten Nebengruppe des Periodensystems verwendet.

5. Verfahren gemäß Anspruch 1 bis 3 unter Verwendung von molekularem Sauerstoff als Komponente a), dadurch gekennzeichnet, daß man als Katalysatorkomponente bb) (i) Oxide und/oder Hydroxide von unter den Reaktionsbedingungen zu Redoxreaktionen geeigneten Elementen der dritten bis fünften Hauptgruppe oder ersten bis achten Nebengruppe des Periodensystems in Kombination mit (ii) anionisch, als Chlorid gebundenes Chlor enthaltenden Verbindungen, die unter den Reaktionsbedingungen zur Aktivierung der Oxide bzw. Hydroxide unter Chloridbildung geeignet sind, verwendet werden.

6. Verfahren gemäß Anspruch 1 bis 4, dadurch gekennzeichnet, daß man als Katalysatorkomponente bb) Eisenoxichlorid verwendet.

7. Verfahren gemäß Anspruch 1 bis 6, dadurch gekennzeichnet, daß man als primäres Amin ein eine oder zwei primäre Aminogruppen aufweisendes aromatisches Amin verwendet.

8. Verfahren gemäß Anspruch 1 bis 7, dadurch gekennzeichnet, daß man als Hydroxylgruppen aufweisende organische Verbindung einen einwertigen, primären aliphatischen Alkohol mit 1 bis 6 Kohlenstoffatomen verwendet.

9. Verfahren gemäß Anspruch 1 bis 8, dadurch gekennzeichnet, daß man die Umsetzung im Temperaturbereich zwischen 100 und 300 °C und bei einem Druck zwischen 5 und 500 bar durchführt.

**Claims**

1. A process for the production of urethanes by reacting primary amines with carbon monoxide and organic compounds containing at least one hydroxyl group, characterised in that the reaction is carried out in the presence of

a) molecular oxygen and/or organic nitro compounds as oxidising agents and

b) a catalyst system consisting of

ba) a noble metal and/or a noble metal compound of the Eighth Secondary Group of the Periodic System of Elements and

bb) compounds — different from the compounds ba) — of elements of the Third to Fifth Main Group and/or of the First to Eighth Secondary Group of the Periodic System of elements which are capable of redox reactions under the reaction conditions ; where organic nitro compounds are used as component a), component bb) is formed by oxychlorides of elements of the Third to Fifth Main Group or First to Eighth Secondary Group of the Periodic System which are capable of redox reactions under the reaction conditions or by combinations of (i) oxides and/or hydroxides of elements of the Third to Fifth Main Group or First to Eighth Secondary Group of the Periodic System which are suitable for redox reactions under the reaction conditions with (ii) compounds which contain chlorine anionically bound as chloride and which are suitable for activating the oxides or hydroxides under the reaction conditions accompanied by chloride formation.

2. A process as claimed in Claim 1, characterised in that the nitro compound constitutionally corresponding to the primary amine used is used as the organic nitro compound.

3. A process as claimed in Claims 1 and 2, characterised in that palladium, rhodium, palladium compounds and/or rhodium compounds are used as catalyst component ba).

4. A process as claimed in Claims 1 to 3 using molecular oxygen as component a), characterised in that oxychlorides of elements of the Third to Fifth Main Group or of the First to Eighth Secondary Group of the Periodic System which are capable of redox reactions under the reaction conditions are used as catalyst component bb).

5. A process as claimed in Claims 1 to 3 using molecular oxygen as component a), characterised in that (i) oxides and/or hydroxides of elements of the Third to Fifth Main Group or of the First to Eighth Secondary Group of the Periodic System which are suitable for redox reactions under the reaction conditions in combination with (ii) compounds which contain chlorine anionically bound as chloride and which are suitable for activating the oxides or hydroxides under the reactioh conditions accompanied by chloride formation, are used as catalyst component bb).

6. A process as claimed in Claims 1 to 4, characterised in that iron oxychloride is used as catalyst component bb).

7. A process as claimed in Claims 1 to 6, characterised in that an aromatic amine containing one or two primary amino groups is used as the primary amine.

8. A process as claimed in Claims 1 to 7, characterised in that a monohydric primary aliphatic alcohol containing from 1 to 6 carbon atoms is used as the organic compound containing hydroxyl groups.

9. A process as claimed in Claims 1 to 8, characterised in that the reaction is carried out at temperatures in the range from 100 to 300 °C and under a pressure of from 5 to 500 bars.

**Revendications**

1. Procédé de production d'uréthannes par réaction d'amines primaires avec l'oxyde de carbone et des composés organiques portant au moins un groupe hydroxyle, caractérisé en ce qu'on conduit la réaction en présence

a) d'oxygène moléculaire et/ou de composés organiques nitrés comme agent oxydant et

b) d'un système catalyseur qui est formé

ba) d'un métal noble et/ou d'un composé de métal noble du huitième sous-groupe du Système Périodique des Eléments et

bb) de divers composés, différents des composés ba), capables de réactions d'oxydo-réduction dans les conditions réactionnelles, d'éléments des troisième à cinquième groupes principaux et/ou des premier à huitième sous-groupes du Système Périodique des Eléments, de sorte que dans le cas de l'utilisation de composés organiques nitrés comme composant a), on utilise comme composant bb) des oxychlorures d'éléments, capables de réactions d'oxydoréduction dans les conditions réactionnelles, des troisième à cinquième groupes principaux ou des premier à huitième sous-groupes du Système Périodique ou des associations (i) d'oxydes et/ou d'hydroxydes d'éléments, capables de réactions d'oxydo-réduction dans les conditions réactionnelles, des troisième à cinquième groupes principaux ou des premier à huitième sous-groupes du Système Périodique avec (ii) des composés contenant du chlore en liaison anionique sous forme de chlorure, qui conviennent dans les conditions réactionnelles à l'activation des oxydes ou hydroxydes avec formation de chlorure.

2. Procédé suivant la revendication 1, caractérisé en ce qu'on utilise comme composé organique nitré le composé nitré qui correspond par sa constitution à l'amine primaire utilisée.

3. Procédé suivant la revendication 1 ou 2, caractérisé en ce qu'on utilise comme composant ba) du catalyseur le palladium, le rhodium, des composés de palladium et/ou des composés de rhodium.

4. Procédé suivant les revendications 1 à 3, avec utilisation d'oxygène moléculaire comme composant a), caractérisé en ce qu'on utilise comme composant bb) du catalyseur des oxychlorures d'éléments, aptes à des réactions d'oxydo-réduction dans les conditions réactionnelles, des troisième à cinquième groupes principaux ou des premier à huitième sous-groupes du Système Périodique.

5. Procédé suivant les revendications 1 à 3, avec utilisation d'oxygène moléculaire comme composant a), caractérisé en ce qu'on utilise comme composant bb) du catalyseur (i) des oxydes et/ou des hydroxydes d'éléments, capables de réactions d'oxydo-réduction dans les conditions réactionnelles, des troisième à cinquième groupes principaux ou des premier à huitième sous-groupes du Système Périodique en association avec (ii) des composés renfermant du chlore en liaison anionique comme chlorure, qui conviennent dans les conditions réactionnelles à l'activation des oxydes ou hydroxydes avec formation de chlorure.

6. Procédé suivant les revendications 1 à 4, caractérisé en ce qu'on utilise l'oxychlorure de fer comme composant bb) du catalyseur.

7. Procédé suivant les revendications 1 à 6, caractérisé en ce qu'on utilise comme amine primaire une amine aromatique portant un ou deux groupes amino primaires.

8. Procédé suivant les revendications 1 à 7, caractérisé en ce qu'on utilise comme composé organique porteur de groupes hydroxyle un alcool aliphatique primaire monovalent ayant 1 à 6 atomes de carbone.

9. Procédé suivant les revendications 1 à 8, caractérisé en ce qu'on conduit la réaction dans la plage de températures de 100 à 300 °C et à une pression comprise entre 5 et 500 bars.